# EUROPÄISCHE PATENTSCHRIFT

(11) **EP 1 443 592 B1**
(45) Veröffentlichungstag und Bekanntmachung des Hinweises auf die Patenterteilung: **09.08.2006**
(21) Anmeldenummer: 03025930.3
(22) Anmeldetag: 13.11.2003
(51) Int. Cl.: H01Q 1/27, H01Q 7/00, H01Q 1/22

(54) **Gürtelspule als Sende-/Empfangsantenne in einer Transpondereinrichtung**
Belt coil as transmit-/receive antenna in a transponder assembly
Bobine-ceinture comme antenne de transmission/réception dans un assemblage transpondeur

(30) Priorität: 22.01.2003 DE 10302550
(43) Veröffentlichungstag der Anmeldung: 04.08.2004
(73) Patentinhaber: Forschungszentrum Karlsruhe GmbH, 76133 Karlsruhe (DE)
(72) Erfinder: Haller, Dirk, 76189 Karlsruhe (DE); Fischer, Harald, 76356 Weingarten (DE)
(74) Vertreter: Rückert, Friedrich

(56) Entgegenhaltungen:
- EP-A- 1 260 176
- DE-B- 1 272 384
- GB-A- 2 198 898
- US-A- 2 101 033
- US-A- 5 883 376
- PATENT ABSTRACTS OF JAPAN Bd. 0070, Nr. 58 (E-163), 10. März 1983 (1983-03-10) -& JP 57 206102 A (SONY KK), 17. Dezember 1982 (1982-12-17)

## Beschreibung

Die Erfindung betrifft eine Gürtelspule als Sende/Empfangsantenne für Transpondersysteme im human- und/oder tiermedizinischen Einsatz.

Für die Messdatenerfassung von medizinisch interessanten physiologischen Parametern, wie Druck, Temperatur, pH-Wert etc., bei Mensch und Tier, ist es oftmals wichtig, diese Daten über längere Zeit und möglichst unter natürlichen Lebensbedingungen zu erfassen, beispielsweise bei einem Langzeit-EKG. Dies wird bisher messtechnisch unzulänglich gelöst, insbesondere dann, wenn die Messgröße aus größeren Tiefen im Innern des Körpers genommen werden soll und nicht über die Hautoberfläche elektrisch ableitbar ist, wie etwa Blasendruck oder Hirndruck. Damit die Messungen unter möglichst normalen Lebensbedingungen durchgeführt werden können, darf die Messeinrichtung die Bewegungsfreiheit, die physiologischen Funktionen und das Wohlempfinden des Patienten nicht wesentlich einschränken. Dies bedeutet, dass die Messeinrichtung am oder besser im Körper tragbar und möglichst wartungsfrei oder wartungsfreundlich sein muss.

Das größte Problem bei solchen Einrichtungen betrifft das Bereitstellen der nötigen Betriebsenergie. Trotz großer Fortschritte in der Minimierung des Energieverbrauchs integrierter Schaltungen, erlauben auch die sparsamsten Mikrochips keine Langzeitmessungen über Zeiträume von mehr als einigen Tagen bis maximal Wochen. Spätestens dann ist die Batterie oder der Akkumulator der Messeinrichtung verbraucht und muss ersetzt werden. Aufgrund der recht kurzfristigen Wartungszyklen solcher Einrichtungen sind sie nicht für eine Implantation in größeren Tiefen des Körpers geeignet, da für jeden Batteriewechsel eine belastende Operation fällig wäre.

Sollen heute über längere Zeiträume Messdaten aus tieferen Regionen des menschlichen oder tierischen Körpers gewonnen werden, greift man auf folgendes Verfahren zurück:
Der eigentliche Messwertaufnehmer, der Sensor, wird an den Ort im Körper, an dem die interessierende Größe gemessen werden soll, implantiert. Die aufgenommene Messgröße wird dann elektrisch mittels Kabel beispielsweise oder mechanisch mittels z.B. eines Druckmesskatheter an eine außerhalb oder knapp unterhalb der Haut implantierte Messdatenverarbeitungseinrichtung weitergeleitet.

Aus der Vergangenheit heraus ist die Tendenz zu beobachten, dass Messdatenverarbeitungseinrichtungen nicht außerhalb des Körpers sondern zunehmen unter der Haut implantiert werden. Die großen Integrationsdichten moderner Halbleiterchips erlauben es, komplexe Funktionen auf einem Chip unterzubringen. Damit sind die Verarbeitungssysteme für viele Anwendungen klein genug, um sie subkutan zu implantieren.

Auch subkutan implantierte Messdatenverarbeitungseinrichtungen müssen energetisch versorgt/angeschlossen werden. Allerdings ist ein Wartungseingriff zum Batteriewechsel wesentlich schonender für den Patienten, da nur eine oberflächliche Verletzung der Haut notwendig ist, um an das Gerät heranzukommen. Da jedoch auch dieses Verfahren nicht geeignet ist, auf Dauer, wie mehrere Monate oder Jahre, durchgeführt zu werden, hat sich die Technik der induktiven Energieeinkopplung durch die Haut zur Aufladung von Akkumulatoren im Messsystem etabliert.

Obwohl mittels der induktiven Aufladungstechnik keine Eingriffe in den Körper mehr notwendig sind, hat diese Lösung noch Mängel. Ein gravierender Nachteil aller Einrichtungen, bei denen Messwertaufnehmer und Verarbeitungselektronik räumlich getrennt implantiert werden, besteht in der Drahtverbindung, die durch verschiedenste Gewebeschichten des Körpers gelegt werden muss, um beide elektrisch zu koppeln. Zum Beispiel muss bei einer Blaseninnendruckmessung die Blasenwand durch den Druckmesskatheter oder die notwendige elektrische Verbindung zum Sensorelement perforiert werden und eine längere Strecke bis zur nächstgelegenen Hautoberfläche, hier Bauchdecke, überwunden werden. Einerseits werden dadurch Gewebebereiche irreversibel wegen Narbenbildung oder Gewebeveränderung um den Durchstichkanal der Verbindung beschädigt, andererseits wird die Drahtverbindung ständigen mechanischen Wechselbelastungen durch die unvermeidlichen Bewegungen des Gewebes ausgesetzt. Dies führt über längere Zeit zur Materialermüdung und damit Drahtbruch, wodurch das Implantat ausgeschaltet wird.

In der Transpondertechnik, auch mit passiven Transpondern, werden die Transpondereinrichtung im allgemeinen über mehr oder weniger große Distanzen von einem tragbaren oder stationären Auslesegerät energetisch versorgt und nachrichtentechnisch ausgelesen. Dazu hat die Transpondereinrichtung eine Sende-/Empfangsantenne, die so in die Nähe des Transponders gebracht werden muss, dass dieser sich in der Reichweite des elektromagnetischen Wechselfeldes befindet. Bei diesen Antennen handelt es sich um Spulen und damit um eine magnetische Kopplung. Diese Antennenspulen können die verschiedensten Bauformen haben: rechteckige oder runde Rahmenspulen, Stabspulen, flache Leiterplattenspulen etc. Allen diesen Bauformen ist gemeinsam, dass sie einerseits starr aufgebaut sind und andererseits aus einer in sich geschlossenen Anzahl von Leiterwindungen bestehen.

Für den Einsatz zur Messung von physikalischen Größen im Körper sind diese Spulen nicht geeignet, da sich Körperformen an vorgesehenen Körperbereichen nicht anpassen, bzw. anlegen. Ist eine Durchstrahlung des Bauchraumes erwünscht, muss die Spule um den Bauch getragen werden, dabei muss ein fester Sitz der Spule gewährleisten sein, um ein Verrutschen zu verhindern. Sie muss aber dennoch flexibel und dehnbar sein, so dass die Atmungs- und Bewegungsfreiheit des Patienten nicht eingeschränkt werden. Weiterhin muss ein einfaches Anlegen und Ablegen der Spule gewährleistet sein. Auch ein Tragekomfort, zumindest weich und flexibel, muss bestehen .Solche Eigenschaften haben Antennenspulen noch nicht.

Eine tragbare Sende-/Leseantenne für eine Anwendung zur Messung von Größen am lebenden Menschen ist *beispielsweise als* ein System zur Messung der Materialbelastung einer Hüftgelenksprothese bekannt. Hier kam ein passiver Transponder im Innern des Prothesenkopfes zum Einsatz. Die Ausleseantenne wurde um den Oberschenkel etwa in Höhe der Leiste befestigt. Zur Befestigung wurde sie in diesem Experiment so eng gewählt, dass sie sich über die Oberschenkelmuskel spannte und dadurch am Ort blieb. Zusätzlich wurde sie mit Klebeband am Oberschenkel fixiert. Die Antenne wies weder größere Flexibilität noch die Möglichkeit zum Öffnen und Schließen auf.

Aus der DE 1 272 384 B ist eine am Körper einer Bedienperson anliegender Rahmenantenne für ein tragbares, nach dem Induktzonssprinzip arbeitenden Sprechgerät bekannt. Die Rahmenantenne ist als Leibgürtel ausgebildet. In einem ein-oder mehrschichtigen Gürtelwerkstoff ist der Antennenleiter in einer gleichsinnig verlaufenden geschlossenen Windung bzw. sind die Antennenleiter in gleichsinnig verlaufenden geschlossenen Windungen eingelegt oder eingebettet. Vom Prinzip her Gleichartiges wird in dem japanischen Patentabstract, Bd. 0070, Nr. 58 (E-163), 10. März 1983 (1983-03-10) -& JP 57 206102 A (Sony KK) , 17. Dezember 1982 (1982-12-17) beschrieben: In einen an einen menschlichen Körper umlegbaren Gürtel ist eine Drahtschleife eingelegt, die bei umgelegtem und geschlossenen Gürtel eine induktive Drahtschleife bildet, die eine Steckvorrichtung zu einem Antennenteil hat.

Mit der Weiterentwicklung der Transpondertechnik, insbesondere der passiven, energetisch nicht eigenversorgten Transponder, hat sich in den letzten Jahren ein Weg aufgetan, die erwähnten Probleme bei der Messung von physikalischen Größen im Körper zu neutralisieren. Passive Transponder beziehen die zu ihrem Betrieb notwendige Energie aus einem sie durchdringenden magnetischen Wechselfeld. Aus diesem erzeugen sie mittels magnetischer Induktion die zu ihrem Betrieb notwendige Spannung. Auch die Datenübertragung findet unter passiver Nutzung des magnetischen Wechselfeldes statt, indem die Daten durch Belastung der Induktionsspule im Transponder auf das eingestrahlte Magnetfeld aufmoduliert werden. Hochintegrierte Mikrochips vereinigen heute die Sensortechnik zur Erfassung und Vorverarbeitung der Messgröße mit den elektronischen Komponenten für den passiven Transponder auf einem Bauteil. Damit ist es möglich, die gesamte Messeinrichtung an die Stelle im Körper zu implantieren, an der gemessen werden soll. Eine Wartung ist nicht mehr notwendig. Ein solcher passiver Transponder könnte so lange ungestört implantiert bleiben, solange seine Funktion besteht.

Daraus ergab sich die Aufgabe, die der Erfindung zugrunde liegt, nämlich eine um und an einen menschlichen oder tierischen Körper anlegbare, magnetisch empfindliche Sende- und Empfangseinrichtung bereitzustellen, mit der ein im Körperinnern implantierter passiver Transponder energetisch versorgt werden und auf die der Transponder ebenfalls über das Magnetfeld nachrichtentechnisch einkoppeln kann. Dabei soll das Tragen der Antenne die normale Bewegung nicht einschränken, allenfalls erträglich einschränken.

Die Aufgabe wird mit einer Gürtelspule gemäß dem Anspruch 1 gelöst.

Die Gürtelspvle als Sende-Empfangsantenne mit einem einlagigen Bandleiter aus mindestens zwei zueinander parallelen Leitern ist wie ein Gürtel um den Körper anlegbar und kann geschlossen und geöffnet werden. Ein vorgesehenes Gebiet am menschlichen oder tierischen Körper kann so anliegend vollständig umfasst werden.

An einer oder mehreren, dem Anwendungszweck zu berücksichtigenden Stellen des Leiterbandes ist ein in Richtung der elektrischen Leiter, d. h. in Längsrichtung des Bandes, elastisches Trägermaterial eingefügt. Auf diesem ist das Leiterband dergestalt befestigt, dass dieses, bei Entspannung des elastischen Trägermaterials, wellenfrontförmig zusammengerafft wird. Dies führt dazu, dass das Leiterband in Längsrichtung dehnbar ist und sich so einem sich in Grenzen ändernden Umfang stets anpassen, bzw. an in Grenzen verschiedene Umfänge angelegt werden kann. Damit ist eine dynamische Längenanpassung des Leiterbandes im angelegten Fall möglich. Die Parallelfixierung der elektrischen Leiter im Trägermedium sorgt dabei dafür, dass sich gleichmäßige Aufwerfungen des Leiterbandes innerhalb der elastischen Zone bilden.

Der Anfang und das Ende eines Bandleiters enden jeweils in einer Steckerleiste. Diese beiden Steckerleisten können geführt ineinander gesteckt und geführt auseinander gezogen werden. Die Enden der elektrischen Leiter sind derart an Stecker oder Buchse oder an Messer- und Blattkontakte kontaktiert, dass beim Stecken das Ende eines Leiters den Anfang den in eine Querrichtung zum Leiter gesehenen unmittelbar nächsten Leiter kontaktiert. Der Anfang des ersten außenliegenden Leiters als auch das Ende des andern außenliegenden Leiters sind bei der einlagigen Gürtelspule von außen frei zugänglich sind.

Im Fall der Gürtelspule als Sende-Empfangsantenne von mindestens zwei übereinander liegenden Bandleitern, sind die Steckerleisten am Bandanfang und -ende entsprechend viellagig. Bei beiden wird neben der leiterversetzenden Anordnung der Stecker oder Buchsen (oder Messer- oder Blattkontakt) für eine Lage der endende, außenliegende Leiter der unteren Spulenlage beim Zusammenstecken mit dem Beginn oder Anfang der drauffolgenden Spulenlage durch Stecken lageversetzend verbunden. Dadurch entsteht durch das Zusammenstecken eine mindestens zweilagige Spule. Durch an den Steckergehäusen eingelassene Führungszapfen wird das pinrichtige Zusammenstecken erleichtert.

In den Unteransprüchen 2 bis 8 sind Ausgestaltungen beschrieben, die das Zusammenstecken sichern und das Tragen als auch den Anschluss der Gürtelspule erleichtern:
Die beiden Enden des resultierenden Bandes sind mit einer lösbaren und wiederverschließbaren Vorrichtung ausgestattet, welche die leitende Verbindung der elektrischen Leiter einrichtet. Aufgrund der Gestaltung des Verbindungselementes bleiben zwei Anschlüsse, ob einlagig oder mehrlagig, nach außen frei, die den Anschluss der Spule an eine Stromversorgung und eine Nachrichtenempfangseinrichtung ermöglichen.
Die beiden Steckerleisten werden beim Zusammenstecken mit einem Verschluss gegen ungewolltes auseinanderreißen gesichert (Anspruch 2). Der Verschluss kann ein Einrastverschluss oder ein Klettverschluss oder ein Gürtelverschluss oder ein Überwurfbügel sein.

In den elastischen, gürtellängsdehnfähigen Längenabschnitten sind die Leiter gleichartig auf einen elastischen Träger vernäht oder verklebt oder vergossen oder verklammert (Anspruch 6).

Aufgrund der leiterversetzenden Weiterführung der Leiter in den gesteckten Steckerleisten bestehen in einem der beiden Steckerleisten zwei von außen zugängliche Kontakte oder in beiden Steckerleisten je ein von außen zugänglicher Kontakt (Anspruch 7). Alternativ: Zur Bestromung der durch das Zusammenstecken gebildeten Spule ist an einer der beiden Steckerleisten zusätzlich ein von außen zugänglicher, zweipoliger Stecker integriert, von dem aus in dieser Steckerleiste die beiden Kontaktdrähte zur Buchse oder zum Stecker des Spulenanfangs und -endes führen (Anspruch 8). Über die zwei Einzelkontakte oder das eine Kontaktpaar erfolgt der Energie- und Datentransfer.

Auf diese Art können physikalischen Größen am lebenden Mensch oder Tier an den verschiedensten Stellen des Körpers kontinuierlich gemessen werden. Möglichkeiten der medizinischen Messung sind:
Messung im Abdomen, Blasendruck, Magendruck - Spule als Gürtel;
Messung im Kopf, Hirndruck - Spule als flexibles, dehnbares Stirnband,
Messung an Armen und Beinen, Belastungsmessung an Gelenkprothesen und Knochen - Spule als dehnbares Band.

Die Erfindung wird im folgenden anhand der Zeichnung weiter erläutert. Die Zeichnung besteht aus 9 Figuren, und zwar:
Figur 1 der einlagige Bandleiterin eine Stoffbahn eingenäht,
Figur 2 der einlagige Bandleiter in einem Polymerband eingegossen,
Figur 3 der längselastische Abschnitt,
Figur 4 der Steckverbinderverschluß,
Figur 5 Anwendungsbeispiel Blasendruckmessung,
Figur 6 die Gürtelspule von vorne,
Figur 7 die Gürtelspule rückseitig,
Figur 8 die Steckersicherung,
Figur 9 die Leiterversetzung.

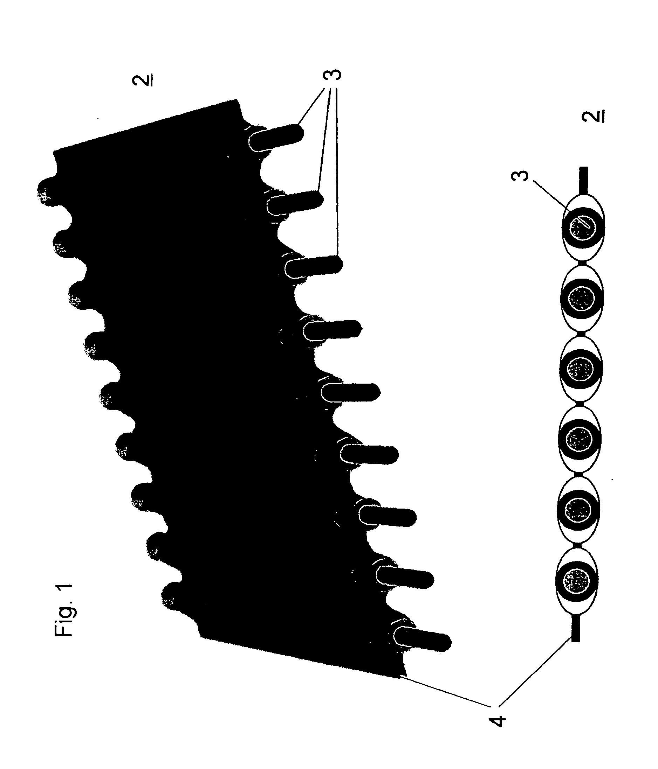

Figur 1 zeigt ausschnittsweise den Aufbau des einlagigen Bandleiters 2. Die parallel nebeneinander liegenden Leiter 3 liegen zwischen zwei Stoffbahnen 4. Beide Stoffbahnen 4 sind außen und entlang der Leiterzwischenbereiche miteinander vernäht und halten so die Leiter 3 in Lage. Statt der Naht können die beiden Stoffbahnen 4 anstelle der Nähte auch verklebt sein. Der Einzelleiter 3 besteht hier aus einer mit einem Mantel elektrisch isolierten Kupferlitze. Aluminiumlitze kommt als Leiter auch in Betracht und wäre insbesondere bei größerer Leiteranzahl wegen des geringen Gewichts in Betracht zu ziehen. Bei entsprechender Wahl des Tuchmaterials bezüglich der elektrischen Isolationseigenschaften ist auch die Verwendung unisolierter elektrischer Leiter denkbar. Damit ergibt sich eine geringere Breite und Dicke des Bandleiters. Alternativ kann somit auch eine höhere Windungszahl der Gürtelspule 1 bei gleicher Breite des Bandleiters erzielt werden.

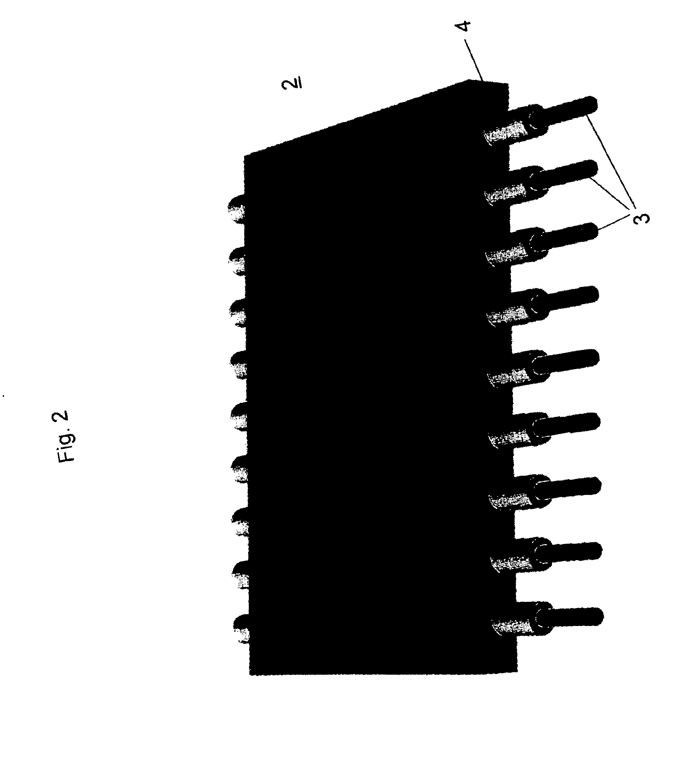

Eine andere Variante des Leiterbands zeigt Figur 2. Hier ist eine Lage parallel zueinander liegender Leiter mit einem Polymer zu einem Band vergossen. Die Leiter 3 selber haben den in der Beschreibung zu Figur 1 erwähnten Aufbau. Dieser Aufbau erinnert eher an ein Gürtelband. Auch hier ist die Verwendung unisolierter Litzen denkbar, um Platz einzusparen, bzw. höhere Windungszahlen zu erzielen.

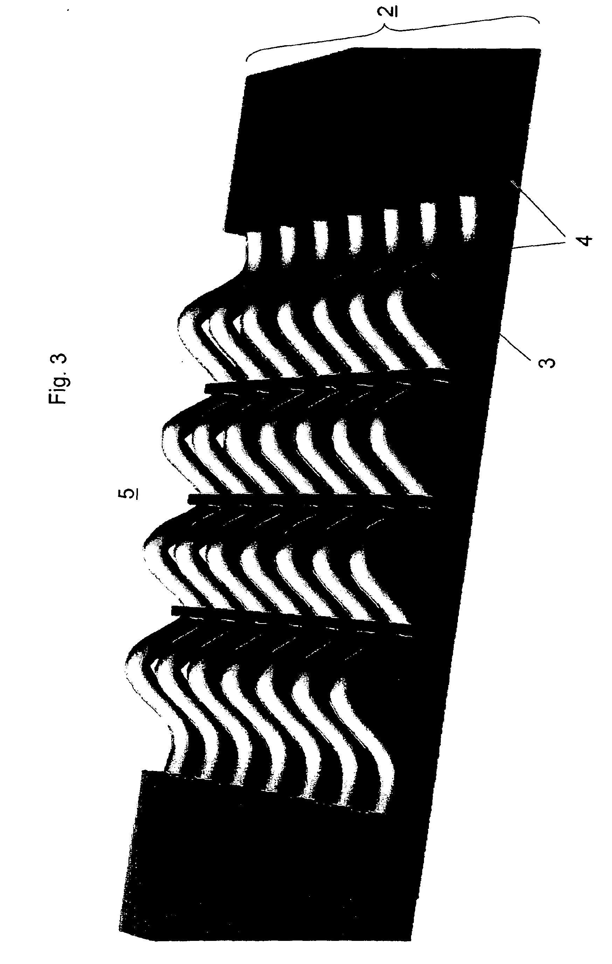

Die abschnittsweise Längselastizität der Gürtelspule 1 zeigt Figur 3 durch Darstellung eines solchen Elastizitätsbereichs 5. Die Darstellung zeigt den entspannten Zustand. Hier ist diese Elastizität durch die gleichgerichtete Wegverlängerung der einzelnen Leiter 3 bei Dehnung des elastischen Trägerbandes realisiert. Die Umweglage der Leiter 3 ist durch Vernähen der Leiter 3 hier in ihren Wellentälern fixiert, kann aber auch durch Verkleben an diesen Stellen durch je einen rechenförmigen Steg erreicht werden. Verklammern an den Auflagestellen der Wellentäler ist ebenfalls denkbar. Insgesamt liegen die Leiter 3 in diesem Abschnitt wie aufeinanderfolgende Wellenfronten.

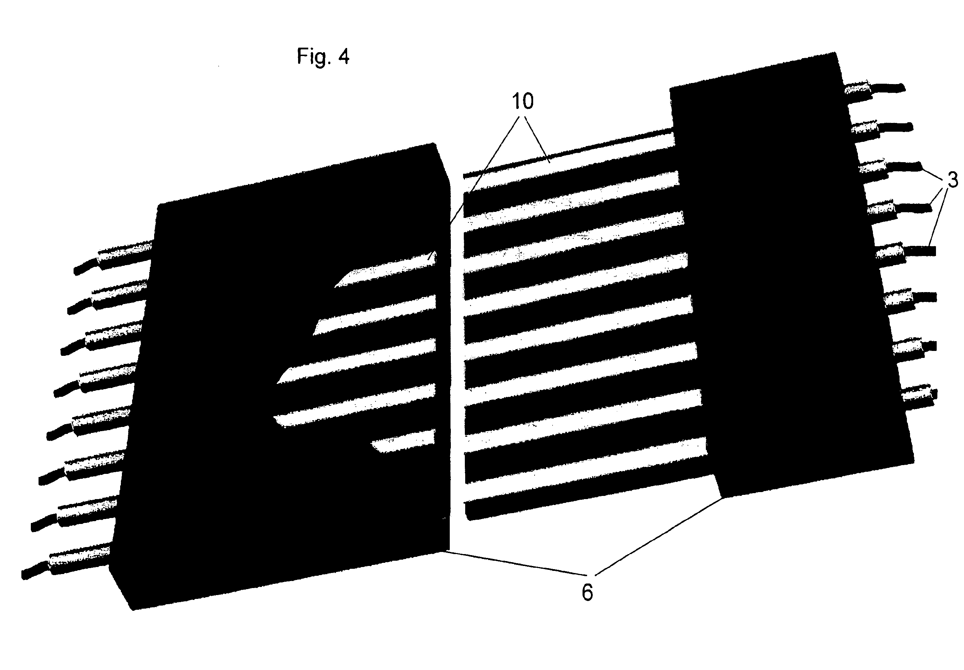

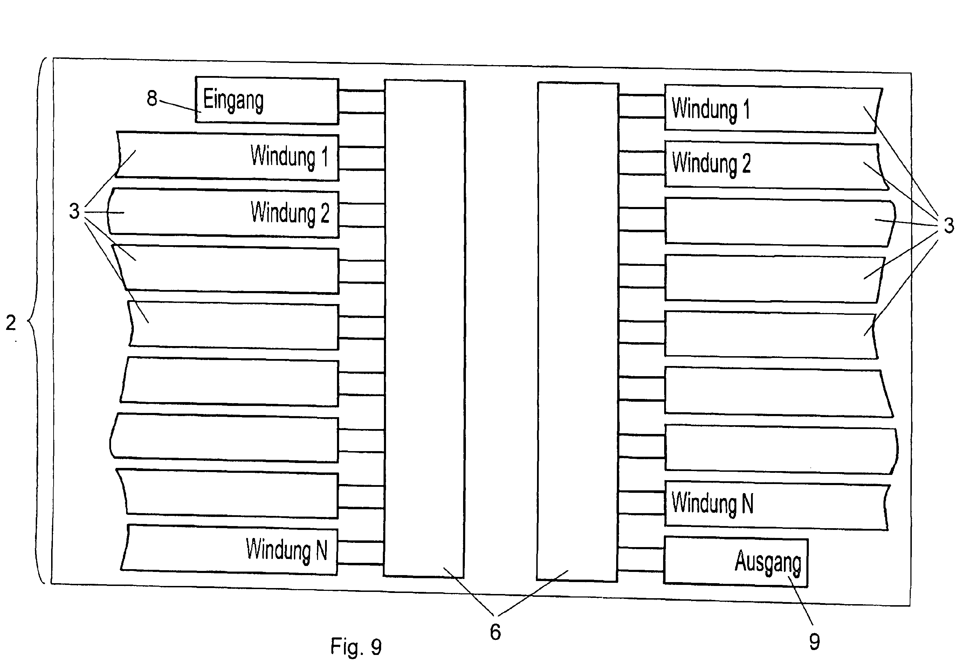

Entscheidend für die Bildung der Gürtelspule 1 aus dem offenen Bandleiter 2 ist der Gürtelverschluss, d. h. die beiden Steckerleisten 6 am jeweiligen Bandleiterende. Figur 4 zeigt beide Steckerleisten 6 für den einlagigen Fall in wenig auseinander gezogener Position. Die Einzelkontakte sind an der im Bild rechten Steckerleiste 3 Messerkontakte 10. Die korrespondierenden Federkontakte sitzen in der im Bild linken Steckerleiste 3, die sich beim Einschieben der Messerkontakte 10 federnd an den zugehörigen Messerkontakt 10 andrücken. Wie die Versetzung, in der Steckverbindung das Leiterende eines Einzelleiters 3 auf den Anfang des nebenliegenden Leiters 3 stößt, ist hier nicht dargestellt. Dies wird in Figur 9 gezeigt. Hier ist die Messerkontakttechnik dargestellt, sie kann aber genauso auch durch die Stecker-Buchsen-Kontaktierung realisiert werden.

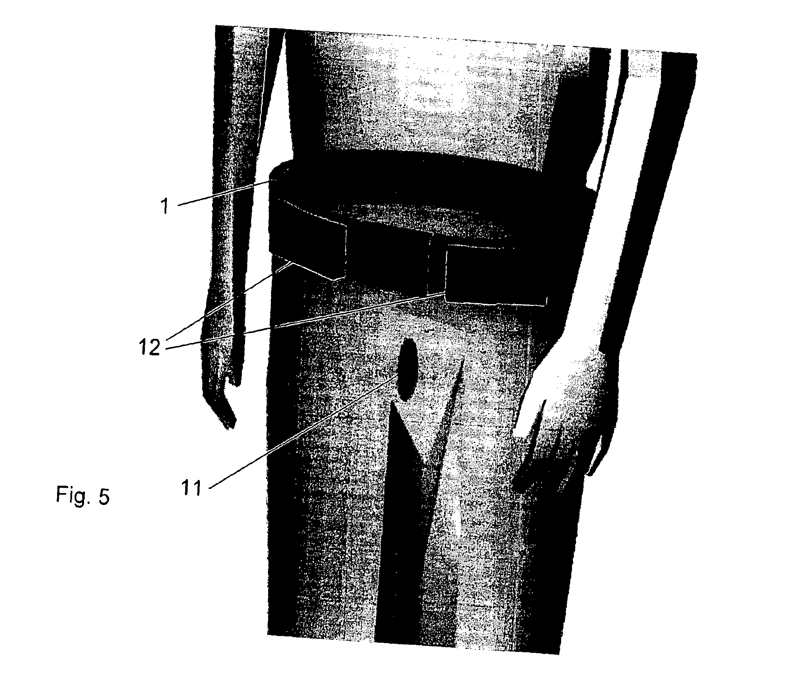

Das Beispiel zur Blaseninnendruckmessung ist in Figur 5 skizziert. Der passive Transponder 11 ist hier durch die im Bild unter der Gürtellinie liegende Ellipse 11 in der Harnblasengegend symbolisiert. Auf der Gürtellinie sitzt die umgelegte und zusammengesteckte Gürtelspule 1. Bei entsprechendem äußerem Design ist sie nicht sofort als medizinisch-technische Einrichtung zu erkennen. Die Darstellung zeigt neben der angedeuteten Verschlussschnalle 7 ebenfalls zwei Einrichtungen 12 links und rechts davon, die auf der Außenseite der Gürtelspule 1 angebracht sind. Dabei handelt es sich einmal um eine mitgetragene Batterie zur Energieversorgung und zum andern um die Steuereinheit zum Auslesen des in der Harnblase sitzenden Transponders 11.

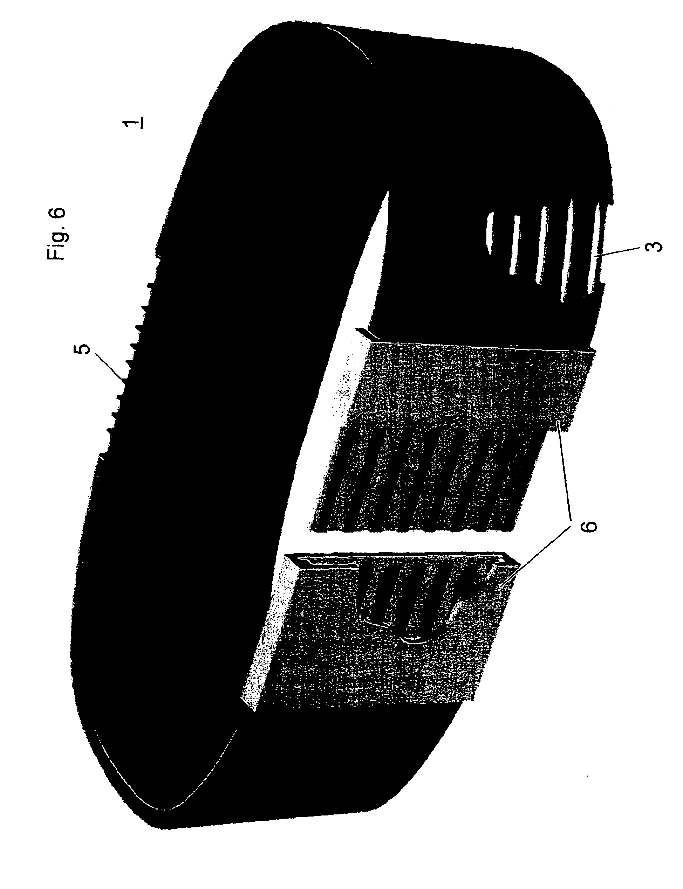

In perspektivischer Gesamtansicht zeigt Figur 6 die Gürtelspule mit ihren drei wesentlichen Merkmalen, nämlich:
erstens die Steckverbindung in Form der beiden nahe beieinanderstehenden Steckerleisten 6,
zweitens den einlagigen Bandleiter 2 und
drittens einen längselastischen Abschnitt 5 auf der Rückenseite.

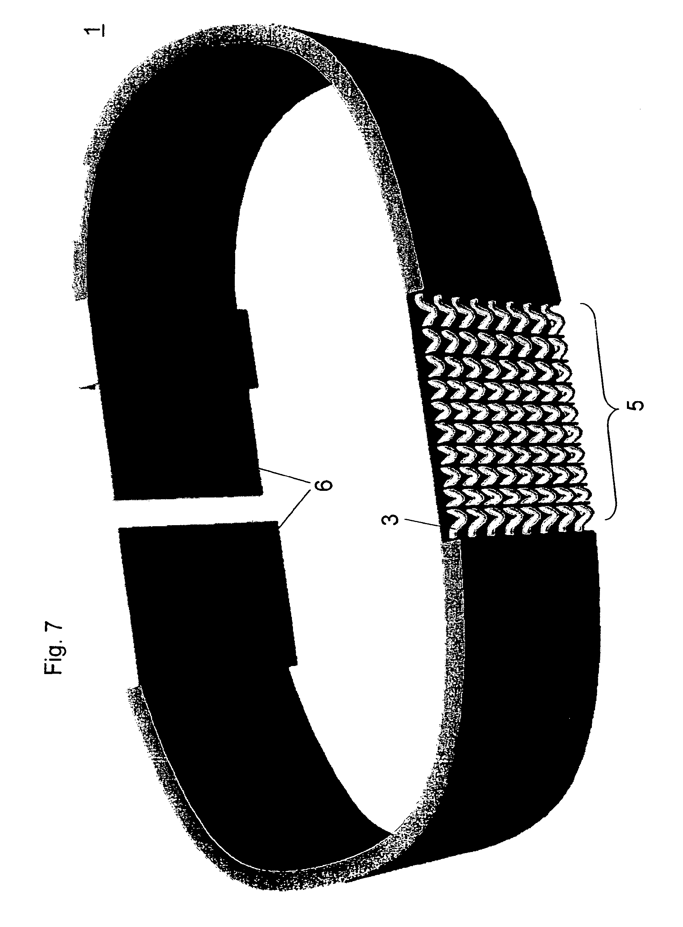

In Figur 7 ist die Gürtelspule 1 von Figur 6 von der Rückenseitensicht her dargestellt, hier ist die wellenfrontenförmige Anordnung der Leiter im entspannten längselastischen Bereich 5 zu sehen.

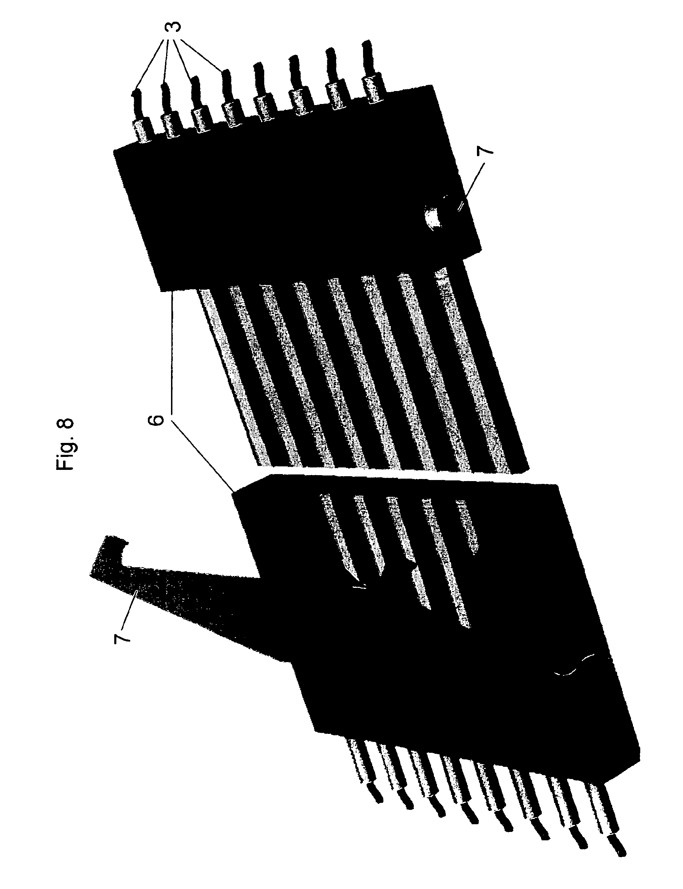

In den Unteransprüchen 2 bis 5 sind zunächst die Sicherung der Steckverbindung und dann die Realisierungsmöglichkeiten beschrieben. In Figur 8 werden die beiden Steckerleisten 6 durch einen an der im Bild linken Steckerleiste 6 drehbar gelagerten Überwurfbügel 7 im zusammengesteckten Zustand gesichert. Der Bügel wird, nachdem die Verbindung zusammengesteckt wurde, auf die im Bild rechte Steckerleiste geklappt, wo er seitlich an dieser an je einem Bolzen 7 mit seiner Nase einrastet. So können sich die Steckerleisten 6 nicht unbeabsichtigt voneinander lösen.

Figur 9 zeigt ein Leiterversetzungsprinzip, das mit einfachen Steckerleisten 6 realisiert wird. Hier liegt ein einlagiger Bandleiter 2 mit n zueinander parallel liegenden Einzelleiter 3 vor. Beide Steckerleisten 3 haben aber (n + 1) Kontakte (n ist eine natürliche Zahl). Der Spuleneingang ist an der im Bild linken Steckerleiste 6 am oberen ersten Kontakt 8 und führt beim Zusammenstecken an der gegenüberliegenden Steckerleiste 6 auf den ersten Kontakt, welcher der Anfang der ersten Windung ist, die an der im Bild linken Steckerleiste auf dem zweiten Kontakt endet, der wiederum in die zweite Windung mündet. Dies Schema wird sukzessive mit allen folgenden Leitern wiederholt, bis die n-te Windung mit ihrem Ende in den Spulenausgang 9 mündet, dies ist der (n + 1)-te Kontakt an der im Bild rechten Steckerleiste 3.

Andere Steckerprinzipien sind aus der Steckertechnik hinlänglich bekannt und wären, falls für diesen Anwendungsfall geeignet, ebenfalls einsetzbar. Herstellungstechnisch ist die dargestellte Technik einfacher Standard und damit wirtschaftlich.

### Bezugsszeichenliste

- 1: Gürtelspule
- 2: Bandleiter
- 3: Leiter
- 4: Trägermedium, Stoffbahn
- 5: Elastizitätsbereich, Längenabschnitt
- 6: Steckerleiste
- 7: Verschluss
- 8: Eingang, Anfang, Spulenanfang
- 9: Ausgang, Ende, Spulenende
- 10: Messerkontakt
- 11: Transsponder, Ellipse
- 12: Einrichtung

## Patentansprüche

1. Gürtelspule als Sende-/Empfangsantenne in einer Transpondereinrichtung für die human- und/oder tiermedizinische Verwendung, bei der die Gürtelspule (1) aus mindestens einem elektrisch isolierten, einlagigen Bandleiter (2) mit einer Länge derart besteht, dass ein vorgesehenes Gebiet des menschlichen oder tierischen Körpers anliegend umfasst werden kann,
ein solcher Bandleiter (2) aus mindestens einem elektrisch isolierten flexiblen Leiter (3) besteht, und
im Fall der Gürtelspule (1) als Sende-Empfangsantenne mit mindestens zwei solchen Leitern (3) diese, elektrisch gegeneinander isoliert, parallel nebeneinander liegen, jeder Bandleiter (2) auf einem zugfesten Trägermedium (4) sitzt,
der Anfang und das Ende eines Bandleiters (2) in je einer Steckerleiste (6) enden und diese beiden Steckerleisten (6) geführt ineinander steckbar und geführt auseinanderziehbar sind, **dadurch gekennzeichnet, dass**: jeder ßandleiter (2) jeweils an mindestens einem gleichen ort einen Längenabschnitt (5)hat, der längs elastisch dehnfähig ist,
wobei die einzelnen Leiter (3) derart auf dem Stecker oder der Buchse der jeweiligen Steckerleiste (6) leiterversetzend kontaktiert sind, daß beim Stecken das Ende eines Leiters (3) auf den Anfang des in eine Richtung quer zum Leiter (3) gesehenen, nächsten Leiters (3) stößt und
der Anfang des ersten außenliegenden Leiters (3) sowie das Ende des andern außenliegenden Leiters (3) bei der einlagigen Gürtelspule (1) im Falle einer einlagigen Spule von außen frei zugänglich sind,
oder
im Fall der Gürtelspule (1) als Sende-Empfangsantenne von mindestens zwei übereinander liegenden Bandleitern (2), die beiden Steckerleisten (6) jeweils entsprechend viellagig sind,
wobei bei beiden neben der leiterversetzenden Kontaktierung der Stecker oder Buchsen für eine Lage der endende, außenliegende Leiter (3) der unteren Spulenlage beim Zusammenstecken mit dem Beginn oder Anfang der drauffolgenden Spulenlage durch Stecken lageversetzend verbunden wird und weiter die Leiter (3) dieser neuen Lage leiterversetzend wie im einlagigen Fall beim Stecken kontaktiert werden
und
der Anfang (8) des ersten außenliegenden Leiters (3) der untersten Lage als auch das beim Stecken erhaltene freie Ende (9) des außenliegenden Leiters (3) der letzten Lage der so zustande kommenden mindestens zweilagigen Spule von außen frei zugänglich sind.

2. Gürtelspule nach Anspruch 1, **dadurch gekennzeichnet, dass** die beiden zusammengesteckten Steckerleisten (6) in ihrer Position durch einen Verschluss (7) gesichert sind.

3. Gürtelspule nach Anspruch 2, **dadurch gekennzeichnet, dass** die beiden zusammengesteckten Steckerleisten (6) mit einem lösbaren Einrastverschluss (7) gesichert sind.

4. Gürtelspule nach Anspruch 2, **dadurch gekennzeichnet, dass** die beiden zusammengesteckten Steckerleisten (6) über einem Klettverschluss (7) zusammengehalten werden.

5. Gürtelspule nach Anspruch 2, **dadurch gekennzeichnet, dass** die beiden zusammengesteckten Steckerleisten (6) mit einem an der einen Steckerleiste (6) drehbar verankerten und am andern einrastbaren Überwurfbügel (7) vor dem unbeabsichtigten Auseinanderziehen gesichert werden können.

6. Gürtelspule nach den Ansprüchen 2 bis 5, **dadurch gekennzeichnet, dass** im elastisch dehnfähigen Längenabschnitt (5) die Leiter (3) auf einem elastischen Träger vernäht oder verklebt oder verklammert sind.

7. Gürtelspule nach Anspruch 6 **dadurch gekennzeichnet, dass** an jeder der beiden Steckerleisten (6) eine von außen zugängliche einpolige Steckvorrichtung eingebaut ist, von der aus ein Kontaktdraht zum jeweils freien Leiterende der Buchse oder des Steckers und damit des Spulenanfangs (8) und -endes (9) führt.

8. Gürtelspule nach Anspruch 6 **dadurch gekennzeichnet, dass** an einer der beiden Steckerleisten (6) eine von außen zugängliche zweipolige Steckvorrichtung eingebaut ist, von der aus in dieser Steckerleiste (6) die beiden Kontaktdrähte zur Buchsen- und zum steckerseitigen Spulenanfang (8) und -ende (9) führen.

## Claims

1. Belted coil as a transmitting/receiving antenna in a transponder device for use in human and/or veterinary medicine, in which the belted coil (1) is made up of at least one electrically insulated single-layer strip conductor (2) of a length such that a designated area of a human or animal body can be closely embraced; a strip conductor (2) of this type consists of at least one electrically insulated flexible conductor (3) and, in the case of the belted coil (1) as a transmitting/receiving antenna with at least two such conductors (3), these conductors are arranged in parallel side by side and electrically insulated from each other, each strip conductor (2) rests on a high-tensile-strength carrier (4), the beginning and the end of a strip conductor (2) terminate in one multi-point connector (6) each, which two multi-point connectors (6) can be plugged into each other in a guided fashion and detached in a guided fashion, **characterized by** each strip conductor (2) having a length (5) in at least one identical place which can be stretched lengthwise elastically, where the individual conductors (3) are contacted on the plug or the socket of the respective multi-point connector (6) in a fashion offsetting conductors in such a way that, in plugging, the end of one conductor (3) hits the beginning of the next conductor (3) as seen in a direction at right angles relative to the conductor (3), and the beginning of the first outer conductor (3) and the end of the other outer conductor (3) in a single-layer belted coil (1) are freely accessible from the outside in a single-layer coil
or,
in the case of the belted coil (1) as a transmitting/receiving antenna of at least two superimposed strip conductors (2), the two multi-point connectors (6) have the corresponding numbers of layers, where in both cases, in addition to the contacting pattern offsetting the conductors of the plugs or sockets for one layer, the terminating outer conductor (3) of the lower coil layer, when connected with the beginning or start of the consecutive coil layer, is connected by plugging in such a way as to offset layers and, in addition, the conductors (3) of this new layer are contacted by plugging so as to offset conductors as in the single-layer case,
and
the beginning (8) of the first outer conductor (3) of the lowest layer as well as the free end (9) obtained in plugging of the outer conductor (3) of the last layer of the coil of at least two layers produced in this way are freely accessible from the outside.

2. Belted coil as claimed in Claim 1, with the two multi-point connectors (6) plugged together being secured in their position by a lock (7).

3. Belted coil as claimed in Claim 2, with the two multi-point connectors (6) plugged together being secured by a detachable lock-down device (7).

4. Belted coil as claimed in Claim 2, with the two multi-point connectors (6) plugged together being joined by a Velcro fastener (7).

5. Belted coil as claimed in Claim 2, with the possibility to protect the two multi-point connectors (6) against inadvertent detachment by means of a hasp (7) pivoted on one of the multi-point connectors (6) and lockable on the other (7).

6. Belted coil as claimed in Claims 2 to 5, with the elastically extensible length (5) of the conductors (3) sewed or bonded or clamped to an elastic carrier.

7. Belted coil as claimed in Claim 6, with a single-pole plug-in device accessible from the outside attached to each of the two multi-point connectors (6), from which a contact wire runs to the respective free conductor end of the socket or plug and thus to the beginning (8) and end (9) of the coil.

8. Belted coil as claimed in Claim 6, with a two-pole plug-in device accessible from the outside attached to one of the two multi-point connectors (6), from which the two contact wires in this multi-point connector (6) run to the socket side and to the plug-side beginning (8) and end (9) of the coil.

## Revendications

1. Bobine à ceinture servant d'antenne émettrice/réceptrice dans un transpondeur destiné aux applications en médecine humaine et vétérinaire, où la bobine à ceinture (1) se compose d'au moins un conducteur plat (2) isolé électriquement, à une couche, d'une longueur telle qu'il est possible d'étreindre d'une manière collante un domaine défini du corps humain ou animal,
qu'un tel conducteur plat (2) se composant d'au moins un conducteur isolé électriquement, flexible, et
dans le cas de la bobine à ceinture (1) utilisée comme antenne émettrice/réceptrice ayant au moins deux conducteurs (3) de ce genre, ceux-ci, isolés électriquement l'un contre l'autre, étant parallèles, chaque conducteur plat (2) étant placé sur un milieu porteur (4) résistant à la traction,
le début et le bout d'un conducteur plat (2) débouchant chacun dans un connecteur multipoint (6), ces deux connecteurs multipoints (6) étant emboîtables l'un dans l'autre d'une manière guidée et enlevables l'un de l'autre d'une manière guidée,
**caractérisé en ce que**
chaque conducteur plat possède au moins à un même endroit une section de longueur (5) extensible à élasticité longitudinale
les différents conducteurs (3) étant chacun contacté à décalage sur la prise mâle ou femelle du connecteur multipoints (6) de telle manière que lors de l'enfichage, le bout d'un conducteur (3) bute sur le début du conducteur suivant par rapport à la direction transversale au conducteur (3) et
le début du premier conducteur (3) extérieur ainsi que le bout de l'autre conducteur extérieur (3) de la bobine à ceinture à couche unique (1) étant librement accessible de l'extérieur dans la cas d'une bobine à couche unique, ou
que dans le cas de la bobine à ceinture (1) utilisée comme antenne émettrice/réceptrice d'au moins deux conducteurs plats (2) superposés, les deux connecteur multipoints (6) possèdent les couches multiples correspondantes,
en plus des contacts à décalage des conducteurs des prises mâles ou femelles pour une couche, le conducteur terminal extérieur (3) de la couche inférieure de la bobine étant, pour les deux, connecté par enfichage lors de l'assemblage avec le début de la couche de bobine consécutive en décalant la couche, et les conducteurs (3) de cette nouvelle couche étant contactés lors de l'enfichage en décalant les conducteurs comme dans le cas à couche multiple,
et
le début (8) du premier conducteur extérieur (3) de la couche la plus basse ainsi que l'extrémité libre (9) du conducteur extérieur (3) obtenue lors de l'enfichage de la dernière couche de la bobine d'au moins deux couches ainsi réalisée étant librement accessible de l'extérieur.

2. Bobine à ceinture selon la revendication 1, **caractérisée en ce que** les deux connecteurs multipoints emboîtés (6) sont bloqués dans leur position par une fermeture (7).

3. Bobine à ceinture selon la revendication 2, **caractérisée en ce que** les deux connecteurs multipoints emboîtés (6) sont bloqués par une fermeture à verrouillage déblocable (7).

4. Bobine à ceinture selon la revendication 2, **caractérisée en ce que** les deux connecteurs multipoints emboîtés (6) sont tenus solidaires par une fermeture velcro.

5. Bobine à ceinture selon la revendication 2, **caractérisée en ce que** les deux connecteurs multipoints emboîtés (6) peuvent être protégés contre leur détachement inopiné.

6. Bobine à ceinture selon les revendications 2 à 5, **caractérisée en ce que** dans la section longitudinale extensible élastique (5), les conducteurs (3) sont recousus ou collés ou agrafés sur un support élastique.

7. Bobine à ceinture selon la revendication 6, **caractérisée en ce que** sur chacun des deux connecteurs multipoints (6), un connecteur unipolaire accessible de l'extérieur est intégré d'où un fil de contact mène à l'extrémité libre respective de la prise femelle ou mâle et par conséquent du début (8) et du bout (9) de la bobine.

8. Bobine à ceinture selon la revendication 6, **caractérisée en ce que** sur l'un des deux connecteurs multipoint (6), un connecteur unipolaire accessible de l'extérieur est intégré d'où, dans ce même connecteur multipoints (6), les deux fil de contact mènent u début (8) et au bout (9) de la bobine du côté des prises femelle et mâle.
